# EUROPEAN PATENT APPLICATION

(11) **EP 4 666 941 A1**
(43) Date of publication of application: **24.12.2025**
(21) Application number: 24755785.3
(22) Date of filing: 16.02.2024
(51) Int. Cl.: A61B 5/097, A61B 5/08, G01N 1/22, G01N 33/497, A61B 10/00

(54) **EXHALED AIR SAMPLE COLLECTOR FOR PRE-DIAGNOSIS OF PATHOLOGIES, AND METHOD FOR USING SAME**

(30) Priority: 16.02.2023 BR 202023002985 U; 09.02.2024 BR 202024002765 U
(71) Applicant: Senna Chelles, Gustavo, São Paulo 04015-070, São Paulo (BR); Romy Hayashi, Miriam, São Paulo 04015-070, São Paulo (BR)
(72) Inventor: Senna Chelles, Gustavo, São Paulo 04015-070, São Paulo (BR); Romy Hayashi, Miriam, São Paulo 04015-070, São Paulo (BR)
(74) Representative: Inchingalo, Simona
(86) International application number: PCT/BR2024/050055
(87) International publication number: WO 2024/168414

(57) **Abstract**

The present Utility Model relates to a breath sample collector and its method of use for the pre-diagnosis of pathologies, said portable, disposable or returnable collector comprising: blowing mouthpiece (1); collection chamber (2); solid and/or liquid and/or gel internal medium for absorption of volatile or non-volatile substances (3); valve (4) that allows air passage but blocks the return of air from the absorption medium; air outlet (5); cap for the air outlet (6); cap for the blowing mouthpiece (7); cap for removal of the absorption medium (8) to obtain the samples; and final seal (21) with tactile feedback and a visual code indicating complete closure.

## Description

### FIELD OF APPLICATION

The present Utility Model relates to a breath sample collector, applicable in a non-invasive and exponentially scalable pre-diagnosis system for pathologies through biomarkers present in a patient's exhalation, particularly cancer, using a portable collector for sample acquisition.

### STATE OF THE ART

For medicine to benefit an ever-growing number of people, it is necessary not only to invest in strategies and means of cure but also in prevention. Prevention is a complex process, as it depends on education and initiative on the part of the patient; it must be encouraged by broad-reaching public and private programs to prevent as many patients as possible from reaching advanced stages, aiming to reduce costs and the suffering resulting from pathologies that can be managed in early stages.

Equipment and technologies are capable of screening pathologies in various ways, accessing tissues, and accurately evaluating their morphology, but at high cost. A lowtechnology examination, such as auscultating the heart with a trained ear, can in a few minutes detect potential cardiac and pulmonary problems, determining whether the patient should undergo expensive, invasive tests or tests that may expose them to radiation.

The motivation behind this project is, in addition to providing a pre-diagnosis means as direct and non-invasive as the stethoscope for serious pathologies such as cancer, to raise awareness of the need for prevention and the possibility of detecting pathologies at a stage in which there is a cure and good survival prospects.

Cancers of the lung, mouth, throat, larynx, pharynx, and stomach account for a high percentage of the most common tumor types, as well as the deaths caused by them.

Lung cancer alone is responsible for approximately 1.76 million deaths per year worldwide, bringing a high cost to health systems, as in 70% of cases the disease is detected only at an advanced or locally advanced stage, when surgery ceases to be a curative option due to tumor extension.

Its diagnosis depends not only on determining the level of smoking or exposure to other risk factors and thus calculating the same, but mainly on imaging exams and subsequent biopsies of suspicious tissues. Imaging exams require highly expensive equipment, not always available in small urban centers, rural areas, or isolated locations, as well as well-trained radiologists or artificial intelligence software not yet widely available or certified. X-ray exams, for instance, fail to detect lung cancer nodules in 25% of cases, according to the Roy Castle Lung Center in the United Kingdom. Imaging exams may expose the patient to incremental radiation loads, preventing frequent testing, in addition to representing high costs.

When diagnosis proceeds to biopsy, after positive indications of tumors detected by imaging exams, the procedure is usually carried out by removing/clinically analyzing a tissue sample from the affected organ and must be performed in a hospital or clinical environment; it also depends on specialized labor, with high hourly cost.

Document WO2013132085 teaches a method and portable sampling device for collecting air comprising biomarkers from a subject's exhaled breath for additional sensorbased analysis, employing a sampling membrane arranged to collect air from said exhaled breath. The device further comprises a detachable tubular element having a lumen or membrane in flow connection with the inlet of the collection device, wherein the tubular element is formed by two opposite parts, each part having at least one deflector so that, when connected, the tubular element comprises at least two deflectors arranged on opposite sides of said lumen to provide a non-straight path through the tubular element.

Document CN108742630 teaches a portable breath nano-sensor array detection device, a portable diagnostic device, and a remote intelligent diagnostic cloud; the portable diagnostic device is connected to the remote intelligent diagnostic cloud via a long-distance communication interface. The portable diagnostic device stores local disease characteristics, and the remote intelligent diagnostic cloud stores a large sample database and updates the large sample database through breath samples collected in real-time to update the local pathology signature database.

Document CN101334399B teaches a portable device for lung cancer diagnosis, characterized by having an air bag, air pump, buffer air chamber, reaction air chamber, image acquisition and processing system, and control system. The air bag, air pump, buffer air chamber, flow sensor, and reaction gas chamber passing through solenoid valves are connected in series; the buffer gas chamber is provided with a humidity sensor, a temperature sensor, and an electric heating device; the reaction gas chamber has a porphyrin sensor; the control system includes an integrated development board PC104 module, a single-chip microcomputer MCU, an LCD touch screen, a GPRS module, and a solenoid valve. Drive module, air pump drive module, electric heating module, temperature acquisition module, humidity acquisition module, micro printer module, flow collection module, and controllable lighting device.

Document WO2020123565A1 teaches a breath test method, characterized by comprising the steps of providing a portable breath analyzer, wherein the portable breath analyzer comprises: (i) a removable mouthpiece; and (ii) a main body, wherein the main body includes a first sensor, a second sensor, a processor, and an electrical circuit, wherein the first sensor comprises ammonia-selective material whose resistivity increases in response to an increase in ammonia gas concentration, wherein the second sensor comprises carbon dioxide-selective material whose resistivity increases in response to an increase in carbon dioxide gas concentration, wherein the electrical circuit operatively connects the first sensor and the second sensor to the processor, and wherein the processor measures the resistivity of the first sensor and the second sensor.

Document WO2010065452 teaches a method for detecting whether an individual has a respiratory disease or monitoring an individual with a respiratory disease, said method comprising receiving, in a portable device with a gas chromatograph coupled to a detector array, ambient air to be inhaled by said subject, wherein the portable device includes the gas chromatograph and detector array mounted replaceably on a substrate of the portable device; filtering, via a volatile organic compound (VOC) filter, the ambient air to remove at least a portion of the background VOC content in the ambient air; further filtering, via a nitric oxide (NO) filter, the ambient air to remove at least a portion of the background NO content in the ambient air; providing, in the portable device, at least a portion of the ambient air to said subject to be inhaled; receiving, in the portable device, the exhaled breath of said subject; contacting the exhaled breath of said subject with the gas chromatograph mounted on the substrate of the portable device; producing, by the detector set coupled to the gas chromatograph and mounted on the substrate of the portable device, an indicator based on the exhaled breath of said individual, the indicator being produced based at least in part on one or more analytes in the exhaled breath of said subject, the indicator being indicative of one or more biomarkers in the exhaled breath; and analyzing, by an analysis circuit disposed in said portable device, said indicator to determine whether said subject has asthma, wherein the determination is made based on a concentration level of NO together with VOC detection from the exhaled breath of said subject.

The document WO2015031617A9 teaches a non-invasive method for detecting lung cancer using a patient's exhaled breath. The method consists of obtaining exhaled gas from the sample in question, wherein the exhaled gas comprises a plurality of carbonyl-containing volatile organic compounds (VOCs). Said plurality of carbonyl group-containing VOCs is selected from the group consisting of 2-butanone, 2-hydroxyacetaldehyde, 3-hydroxy-2-butanone, 4-hydroxy-2-hexenal, and a mixture of C5H10O compounds including 2-pentanone and valeraldehyde.

Document WO2010031788A1 relates to a method for diagnosing chronic obstructive pulmonary disease (COPD) by detecting volatile organic compounds (VOCs) in exhaled air, through the identification of markers for disease conditions related to COPD. For COPD diagnosis, there must be detection of the presence of at least two VOCs selected from a group of six VOCs in the exhaled air, which may be isoprene, C16 hydrocarbon, 4,7-dimethylundecane, 2,6-dimethylheptane, 4-methyloctane, and hexadecane.

Document EP3795983A1 Document EP3795983A1 teaches a lung cancer diagnosis method and system based on exhalation and includes: a step of preparing a surfaceenhanced Raman spectroscopy (SERS) substrate; a step of eluting volatile organic compounds (VOCs) included in each of a plurality of cells, providing each of the cell VOC eluates to the SERS substrate and then measuring each of the cellular SERS signals; a step of learning the signal pattern of each cell by applying deep learning to each of the cellular SERS signals; a step of collecting the patient's exhaled gas and liquefying it using silicone oil, providing the liquefied exhaled gas to the SERS substrate and measuring an exhalation SERS signal, then identifying the exhaled gas signal pattern by analyzing the exhalation SERS signal through the deep learning result; and a step of comparing and analyzing the signal pattern of each of the cellular SERS signals and the signal pattern of the exhalation SERS signal, thereby identifying the cellular SERS signal with the greatest similarity to the exhalation SERS signal and confirming and notifying whether a lung cancer cell is present or not based thereon.

Document US2017035326A1 teaches a device comprising an inlet for accepting a sample of ambient air; a disposable mouthpiece for use by a human subject; a sensor array comprising one or more sensors for measuring physical parameters in exhaled breath; an exhaled air sampler for capturing a predetermined volume of exhaled breath; a concentrator for receiving the predetermined volume of air from the exhaled air sampler, the concentrator being configured to collect volatile organic compounds (VOCs) from the predetermined volume of air; and an ionic liquid collector comprising a container configured to hold a volume of at least one ionic liquid, receive the VOCs from the concentrator, and be removed from the device for VOC analysis, wherein the VOCs are adsorbed on a surface of said at least one ionic liquid.

The state of the art teaches devices, some portable, for capturing the exhaled air of a human being or patient for pathology detection, which may be through DNA extraction or other types of biological material, or even capture of biomarkers in the form of VOCs (volatile organic compounds), with various configurations, possibly comprising filtering systems, sensors, nano sensors, temperature measurement devices and other measures, or instant analysis, or connected to information clouds, etc. However, no invention presents a sustainable, disposable or reusable solution, widely accessible in terms of cost and practicality for use in mass public health, compact and intuitive, not dependent on software and hardware, simple and efficient, capable of being employed in locations with less or more infrastructure and human development, with chemical-mechanical structures capable of retaining volatile organic components from exhaled air and preserving them for at least 7 days until analyzed in molecular identification equipment, with collection being performed by the user themself remotely, **outside a clinical environment and without professional assistance,** supported by an application available on a cell phone or smartphone, easily understood, which guides the best way of exhaling, ensuring reliable collection results; after processing and identifying biomarkers in the laboratory using samples from the collector, data analysis is performed through artificial intelligence, and the pre-diagnosis system provides indications about the type and stage of the pathology for medical and public health decisions.

### OBJECTIVES OF THE UTILITY MODEL

In this regard, the objective was to develop a support system for the pre-diagnosis of pathologies, especially cancer, its types and stages, based on disposable or reusable (with cleaning and sterilization) collectors of exhaled breath samples, with subsequent processing by hardware and software, capable of:
- Enabling the rapid, non-invasive and painless capture, preservation, transport, and subsequent analysis of the sample, with qualification and quantification of possible pathological or tumoral biomarkers present in the volatile organic components of the exhalation.
- Intuitively allowing use by untrained users outside clinical-hospital centers, providing low-cost remote analysis with a gold standard (the term commonly used in the medical field to designate the best technological or scientific method currently available to solve a given problem) from residences far from diagnostic facilities.
- Employing machine learning (artificial intelligence) to process quantitative and qualitative data on pathological or tumoral biomarkers and the patient profile, delivering a risk index or a numerical parameter that allows the physician to identify the stage, in order to support their decision regarding the need for other, more invasive confirmatory diagnostic methods.

### PROBLEM SOLUTION AND UNEXPECTED EFFECT

The pre-diagnosis system for pathologies, according to the Utility Model, is of sufficiently low cost and simplicity that it can be used by laypersons or those without exhaustive training, as well as by extensive public health policy programs. This objective is achieved through the absence of electronics and software in the device described herein, the use of resources already in the user's possession (smartphone + application) and by laboratories (mass spectrometers), and by its intelligent mechanical design employing structures, chambers, and absorbent multi-materials to capture, transport, store, and dispense biomarker samples for analysis.

The present Utility Model represents a collector for use in a non-invasive, low-cost, and painless pre-diagnosis system for pathologies, especially cancers and other pathologies related to the digestive and respiratory systems, their types and stages, based on a collector/protector/transporter/dispenser of exhaled breath samples, associated with software algorithms capable of quantifying, qualifying, and transforming the information from the sampled biomarkers into a risk index, assisting medical decisions and allowing pre-diagnosis processes to be performed from residential environments or outside hospital settings.

The present Utility Model represents a sustainable solution, composed of recyclable and separable materials, with low environmental impact and zero toxicity, compatible with absorbent multi-materials, simple and efficient, capable of being employed in less developed or even developed locations, with a structure capable of retaining exhaled air for at least 7 days until analyzed in a location with clinical analysis equipment. The quality of the sample collected by the user themself is ensured both by the mechanical design and by the use of an application available for cell phone or smartphone, easily understood, which will guide the user on the best way to exhale, to ensure high-quality collection for obtaining reliable results, and further provide, through artificial intelligence, indications regarding the stage of the pathology.

### BRIEF DESCRIPTION OF THE FIGURES

Attached are figures as follows:
**Figure 1****:** schematically shows the basic components of the collector with internal liquid medium;
**Figure 2****:** schematically shows the basic components of the collector with solid internal medium;
**Figure 3****:** schematically shows the basic components of the collector with hybrid internal medium (solid and/or liquid and/or gel);
**Figure 4****:** illustrates the basic process of using the collector, or the collection assistance system;
**Figure 5****:** indicates the application support to assist the patient in correct collection, showing a screen with a colored bar;
**Figure 6****:** represents the actual analysis result of a sample from a patient with lung cancer, using a collector with solid medium according to the Utility Model;
**Figure 7****:** represents a mechanical detailing of the device for solid absorbent medium;
**Figure 8****:** shows the flow of air exhaled by the patient through the mouth orifice and throughout the system;
**Figure 9****:** represents the sealing and protection operation of the vial for transport to the laboratory, immediately after the sample collection performed by the patient;
**Figure 10****:** represents the use of the set for laboratory analysis, with a typical sampling needle from mass spectrometers (24);
**Figure 11****:** represents a mechanical detailing of the device aimed at multi-media, which may be liquids, solids, and gels;
**Figure 12****:** represents the operational process of the device in three stages: closed before testing or use (34), open for testing or use (35), and closed again for transport and analysis (36);
**Figure 13****:** represents the open (39) and closed (37/38) states of the device according to the invention;
**Figure 14****:** represents the moment of analysis, using a typical mass spectrometer needle (24);
**Figure 15****:** schematically represents the process of constructing the data analysis algorithm by artificial intelligence, including training (65/66), front-end implementation (67), and data analysis and processing (68/55).

### SUMMARY DESCRIPTION

The present Utility Model describes a breath sample collector and its method of use for the pre-diagnosis of pathologies, said disposable or returnable portable collector comprising: blowing mouthpiece (1); collection chamber (2); solid and/or liquid and/or gel internal medium for absorption of volatile or non-volatile substances (3); valve systems that allow air passage but block air return from the absorption medium; air outlet (5); air outlet occlusion (6); blowing mouthpiece occlusion (7); occlusion against accidental removal of the absorption medium (8) to ensure the samples are obtained and preserved; final seal (21) with tactile feedback and a visual code indicating complete closure.

### DETAILED DESCRIPTION

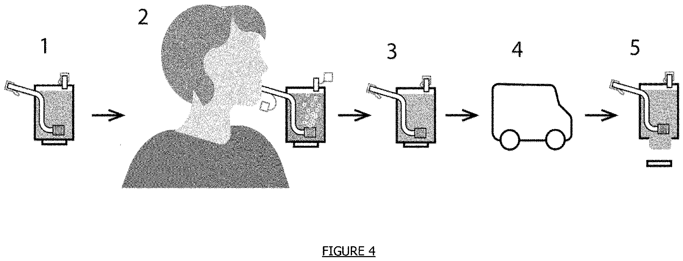

The present Utility Model relates to a breath sample collector for the pre-diagnosis of inflammatory, infectious, and cancerous pathologies, or others that emit volatile organic compounds (VOCs) or may emit biomarkers through exhalation. It is a portable exhaled air collector from the lungs, and a pre-diagnosis system for pathologies including collection, transport, storage, and remote analysis of a patient's exhalation sample using the gold standard method for biomarker detection, as exemplified in Figure 4.

The pre-diagnosis system for pathologies such as cancers of the digestive and respiratory systems is capable of identifying biomarkers that may be eliminated through human exhalation, with data processing via artificial intelligence to determine the numerical degree of risk or disease stage.

Recent scientific research shows that, unlike normal cells, cancer cells release specific substances called tumor biomarkers. When these cells are in contact with air, as in the lungs, they emit the so-called VOCs (volatile organic components). Research has proven that it is possible, in a laboratory environment, to capture these VOCs from exhaled breath, using mass spectrometers to filter and detect the molecules, with high precision and reliability.

This type of pre-diagnosis is non-invasive and has the potential to offer low cost and ease of application, provided it does not depend on being performed in a clinical-hospital environment.

The object of the present Utility Model consists of a breath sample collector for use in a system designed to capture, store, and transport VOCs from a patient's pulmonary exhalation sampled remotely outside a clinical environment, analyze them by MS (mass spectrometry) or electrochemical detection devices, identify and quantify biomarkers, and finally determine a risk level using artificial intelligence algorithms.

The breath sample collector for pathology pre-diagnosis is compact, portable, equipped with multiple chambers, and compatible with multiple absorption media, disposable or returnable, comprising: blowing mouthpiece (1); collection chamber(s) (2); solid and/or liquid and/or gel internal medium for absorbing volatile organic substances (3); valve (4) that allows exhaled air passage but blocks return from the absorption medium; air outlet (5); sealing and closing systems for sample preservation, prevention of improper removal of the absorption medium, and prevention of contamination (6), so that biomarker samples related to the pathologies are collected and sealed easily and intuitively by the patient themself (36), with visual and tactile feedback assistance (21), outside clinical or laboratory environments and without the need for healthcare professionals, ensuring they are properly protected for transport, temporary storage, safe access (5), and remote processing in a specialized laboratory (24), providing raw data for interpretation by an artificial intelligence algorithm (68) that delivers a risk level as pre-diagnostic information for medical or public health decisions, aiming at patient monitoring or further investigation.

According to one embodiment of the Utility Model, the pre-diagnosis system comprises:
- breath sample collector, affordable, allowing the capture of exhaled air samples from the lungs in a non-hospital environment, which may be a residence, a doctor's office, or a small clinic, easily usable by a layperson or healthcare professionals. It features a system for preserving and stabilizing VOCs for a sufficient time so that it can be transported from remote locations, with little or no diagnostic equipment infrastructure, to an analysis center equipped with an MS (mass spectrometry) device or an electrochemical identifier;
- mobile application-based system that guides the step-by-step of the test and is capable of measuring, through signal filtering or Artificial Intelligence, the effectiveness, duration, and strength of the breath, ensuring that the sample is indeed effective and captures the deep gases of the expiratory process.
- chemical analysis system for determining the molecular composition of the sample, which may involve a mass spectrometer or another type of equipment with the same analytical capacity and degree of reliability; and
- artificial intelligence analysis system of biomarkers together with the patient's personal profile, determining the risk level for the different stages of the pathology.

### THE COLLECTOR

The portable breath sample collector comprises blowing mouthpiece (1); collection chamber (2); solid and/or liquid and/or gel internal medium for absorption of volatile or non-volatile substances (3); valve (4) that allows air passage but blocks return from the absorption medium; air outlet (5); cap for the air outlet (6); cap for the blowing mouthpiece (7); cap for removal of the absorption medium (8) to obtain the samples.

Said collector may internally comprise one or more chambers with absorption media for substances from the captured air, presented individually or combined.

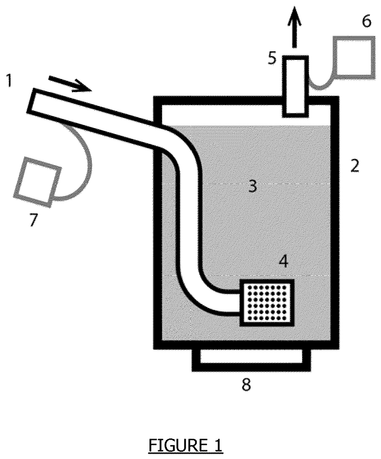

The breath sample collector may be of four types: comprising a liquid absorbent medium, a solid absorbent medium, a solid and liquid absorbent medium (hybrid), or compatible with multiple absorbent media, as illustrated in Figures 1, 2, 3, and 13.

The collector with liquid or gel absorbent medium comprises a container as shown in Figure 1, comprising: blowing mouthpiece (1); collection chamber (2); liquid or gel internal medium for absorption of Volatile Organic Components (3), consisting of substances that retain gases, monomolecular or with known uncontaminated composition, to be subtracted in the analysis phase, such as distilled water and powdered activated carbon dissolved, activated carbon granules, or also hydrogel or aerogel. Furthermore, valve (4) that allows air passage, but blocks return from the absorption medium; air outlet (5); cap for the air outlet (6); cap for the blowing mouthpiece (7); cap for removal of the absorption medium (8) to obtain the samples.

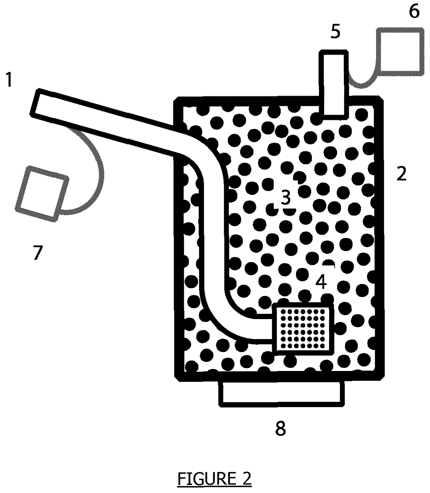

The collector with solid absorbent medium consists basically of a container with the structure shown in Figure 2, namely: blowing mouthpiece (1); collection chamber (2); solid internal medium for VOC absorption (3), characterized by substances that retain gases, monomolecular or with known uncontaminated composition, to be subtracted in the analysis phase. For example, distilled water with absorbent solutes, powdered or granulated activated carbon, hydrogel, aerogel, calcium hydroxide and sodium hydroxide, graphene sheets, MOFs (metal-organic frameworks), porous polymers may be used. Furthermore, valve (4) that allows air passage, but blocks return from the absorption medium; air outlet (5); cap (6) for the air outlet; cap (7) for the blowing mouthpiece; cap for removal of the absorption medium (8) to obtain the samples.

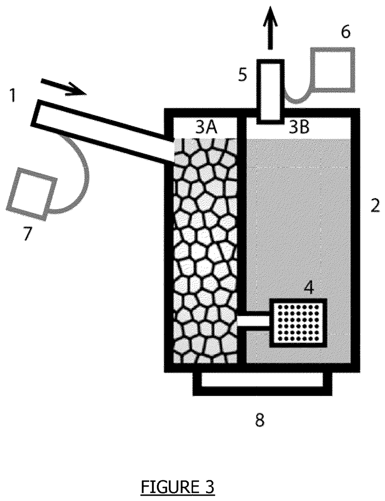

The collector with hybrid absorbent medium (solid and/or liquid and/or gel) consists basically of a container with the structure shown in Figure 3, attached, comprising one or more internal chambers to increase the absorption potential, combining, for example, the characteristics of solid and liquid or gel media, and comprising: blowing mouthpiece (1); collection chambers (2); solid absorption medium (3A); liquid or gel absorption medium (3B). There may be additional passage chambers beyond these two, in order to maximize sampling. It further comprises: valve allowing gas passage (4) but blocking return from the liquid, gel, or solid absorption medium. Examples of the valve (4) include a porous filter element made of polymer, metal, or ceramic. Also includes air outlet (5); cap for the air outlet (6); cap for the blowing mouthpiece (7); and cap for removal of the absorption medium (8) to obtain the samples.

The preservation of VOCs depends on sealing the samples so that the gases contained in the chamber remain inside it, saturating the absorption media by molecular diffusion. Tests carried out by the Applicant have shown that if the storage time is less than 7 days, and the absorption media are inert and pure, there is no sufficient degradation of VOCs to impair the detection and identification of biomarkers. Seven days are compatible with domestic logistics even in a country of large dimensions, so that locations without the necessary resources can be served.

### BREATH SAMPLE COLLECTOR WITH MULTI-ABSORBENT MEDIA

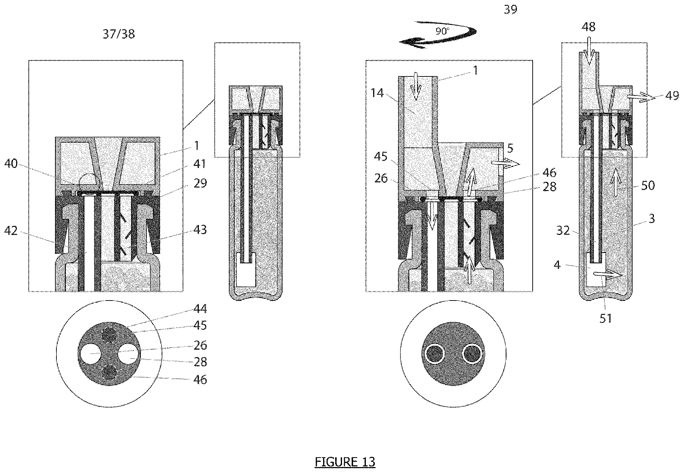

The breath sample collector for pathology pre-diagnosis comprises a disposable or non-disposable collector, of low cost so that it is widely available or can support broadspectrum public policy programs, using liquid absorbent media, or gel (Figure 1), or solids (Figure 2), as well as hybrids and their combination in more than one chamber (Figure 3). The collector using liquid medium (Figure 1) is characterized by a blowing mouthpiece (1) with a seal (7), an air outlet (5) also with a seal (6), a containment container (2) for the liquid or gel absorbent medium (3), and a non-return filter preventing it from returning to the mouthpiece (4). The collector with solid medium (Figure 2) is characterized by a blowing mouthpiece (1) with a seal (7), an air outlet (5) also with a seal (6), a containment container (2) for the solid absorbent medium (3), and a non-return filter preventing it from returning to the mouthpiece (4). The collector with hybrid medium (Figure 3) is characterized by a blowing mouthpiece (1) with a seal (7), an air outlet (5) also with a seal (6), two or more containment containers (2) for the absorbent media (Figures 3A, 3B, etc.), and a non-return filter preventing them from returning to the mouthpiece (4). The multi-media collector (Figure 13) is characterized by a blowing mouthpiece (1) with a sealing and locking system for sample preservation (45/46/26/28), an air outlet (5), a containment container (2) for more than one type of absorbent medium (3) which serves as the actual analysis vial and thus requires no external casing, and a non-return filter preventing them from returning to the mouthpiece (4).

According to one mechanical embodiment of the present invention, the breath sample collector for solid absorbent medium comprises a blowing mouthpiece (1) mounted together with a base (13), protecting an analysis vial (2), a movable needle (18) with an upper cap (6) having a weakened region (9) by thickness reduction or by deposition of thermoplastic rubber membrane. The interface between the blowing mouthpiece (1) and the base (13) has a thread (12) allowing assembly, controlled variation of the distance between them, and sealing of the set after collection.

The vial (2) is dimensioned for the metal trays of common spectrometry equipment (such as GCMS and LCMS). The vial is manufactured from inert material, not emitting Volatile Organic Compounds (VOCs) at the temperature of use and analysis (100 degrees Celsius), and may be made of glass, metal, or thermoplastic resins (polycarbonate, polypropylene, or others), sterilizable for reuse or recyclable.

As the needle (18) and its cap (6) have through holes, as does the inner skirt of the blowing mouthpiece (17), the air exhaled by the patient through the mouthpiece opening (1) fills the first cavity, and the exposed holes in the needle cap allow its passage to the second cavity (18), inside the needle (18), with the air passing through the absorbent medium (3) and then to the next cavity in the circuit (15/16), which communicates with the outside through the air outlet opening (5).

The holes allow air passage and prevent the return or exit of the solid absorbent material in granules, which may be coconut-fiber activated carbon grains with high porosity and purity, particle size of 1 to 2 mm. It is desirable that the absorbent medium be mono-material, so as not to generate interference in the detected molecular spectrum, and neutral so as not to interact with the Volatile Organic Compounds (VOCs) or with the patient's health.

### ON THE SEAL AND PROTECTION OF THE VIAL FOR TRANSPORT

The rotation (23) of the blowing mouthpiece (1) brings the threads between it and the base (12) closer together, pressing the cap (6) against the needle (18), which also moves downward, embedding into the neck of the vial (20). At the end of the stroke, there is a seal (21) with tactile feedback and a visual code indicating complete closure.

This reconfiguration prevents the absorbent medium (3) from contacting the external environment, preserving the volatile biomarkers inside the vial, sealed jointly by the cap (6) and the needle (18), through radial pressure and contact between surfaces (19 and 20). At the end of the rotation stroke, there is a seal (21) with tactile feedback for greater sample and patient safety, preventing reverse rotation unless the seal wall is broken with a tool or by reverse rotation using force. There is also a visual code, which in full closure has the two halves coinciding, allowing the application to recognize that the closure was performed correctly.

### DEVICE FOR MULTI-ABSORBENT MEDIA

The device according to the invention may comprise multiple media, which may include liquids, solids, and gels.

According to another embodiment of the invention, the multi-absorbent medium collector comprises: a vial (2) of glass, over which a cap is applied, consisting essentially of two main parts: the rotatable blowing mouthpiece (1) mounted on the base (13), wherein the inlet air passage opening (26) of the blowing mouthpiece to the cannula (32) directs the exhaled air, rich in volatile biomarkers, to the atomizer or micro-bubble generator (4) and reverse flow retainer; rotation ring and connection between the blowing mouthpiece and base (27); deflection duct to prevent the exit of liquid or gel (31); liquid, gel, or solid medium (3); flange for retaining the cap on the vial neck (30); thermoplastic rubber piece for compression sealing (29), a classical hydraulic sealing solution with O-rings; outlet air passage opening from the absorbent cavity (28); air outlet opening from the device (5).

The vial (2) may be made of glass, metal, or thermoplastic resins (polycarbonate, polypropylene, or others), sterilizable for reuse or recyclable, manufactured from inert material that does not emit Volatile Organic Compounds (VOCs) at the temperature of use and analysis (100 °C).

The atomizer or micro-bubble generator (4) and reverse-flow retainer may be made of sintered ceramic or high-porosity polymers.

The operational process of the device has three stages: closed before testing (34), open for testing (35), and closed again for transport and analysis (36). The transition between each of these three stages is carried out by rotating the blowing mouthpiece clockwise in 90-degree steps, aided by a tactile marker and by mechanical stops in the rotation ring. These structures prevent a return to the open position when the patient closes the system immediately after the test and ensure sample integrity, aligned with the visual marking for the patient (33).

To close the device after testing, the user rotates it another 90 degrees clockwise so that misalignment and occlusion occur again (40/41), preserving the sample. In the open state (39), the blown air (48) passes through the blowing mouthpiece (1), fills the cavity (14), passes through the passage holes (45 aligned with 26), enters the absorption cavity via the cannula (42) reaching the atomizer or micro-bubble generator (4), passes (51) into the absorbent medium (3), flows vertically (50), passes through the passage holes to the outlet (46 aligned with 28), and finally exits (49) the device through the outlet opening (5).

The system and collector, according to the Utility Model, guide the user through the steps to be performed via an application; the system includes a mobile phone or smartphone application which, in addition to step-by-step guidance, explains the process and has a sound-processing algorithm using the device's microphone, capable of interpreting the strength, frequency, and duration of inhalation and exhalation (3). A bar graphically shows the intensity and correct duration through amplitude and color, turning green when approaching the ideal, and remaining red otherwise.

### THE SOFTWARE APPLICATION

The software application explains how capture works and how the system operates; it instructs the user on how to handle and use the device; assists the user in inhaling and exhaling correctly to maximize test success; and informs how to seal the sample inside the device and how to send it for analysis.

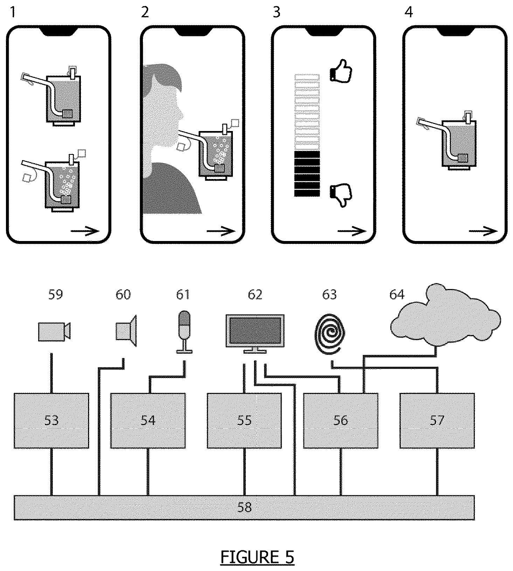

The pathology pre-diagnosis system is assisted by a smartphone application, which, in addition to guiding the collection procedure step-by-step, also helps the user maximize inhalation and exhalation to provide the best possible sample, measuring their intensity and duration through sensing, and displaying this using graphical resources (Figure 5).

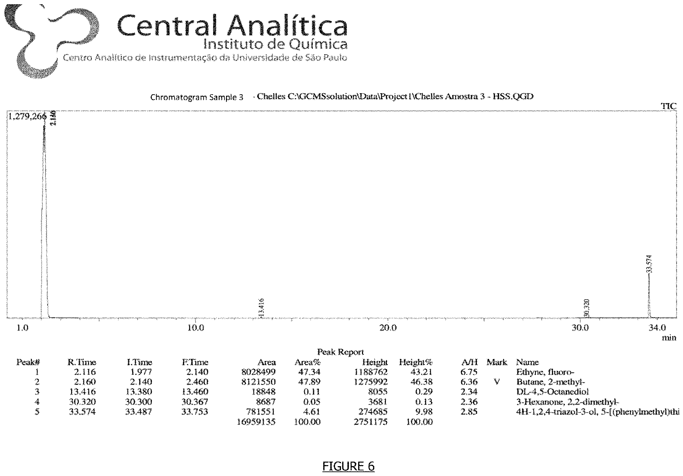

The pathology pre-diagnosis system according to the Utility Model uses mass spectrometry equipment (Figure 6) to detect biomarkers, but may also employ other molecular identification technologies which, if miniaturized and made cost-effective while maintaining the gold standard of reliability, could be integrated into the collectors, providing immediate pre-diagnosis.

The pre-diagnosis system is supported by software developed for both PC and mobile devices, with its logical structure described in Figure 5. The algorithm uses the camera (59) to read the bidimensional code of the purchased test kit, thereby synchronizing patient data with the kit. System access, for both patient and physician, is controlled through an access controller and biometric authentication, which may use a biometric reader (63) or the device's camera (59). A digital assistant (58) guides the user through system navigation step-by-step, showing animations and videos (62) and spoken instructions (60), ensuring accessibility for people with disabilities and the elderly. As shown in Figure 5, step 3, the sampling-support intelligence module uses the microphone (61) to detect blowing noise, its intensity and duration, providing graphic (62) and audio (60) feedback regarding compliance with the minimum parameters for a good sample, asking the user to repeat the blow if necessary. A logistics control module for analysis (56) allocates the necessary partners to transport and identify the biomarkers, releasing raw data to the cloud-based artificial intelligence processing system (64). Finally, the results organization module (55) provides a graphical and parametric display of the risk level, in addition to storing historical results for physician analysis. The reason for providing a risk level rather than a POSITIVE or NEGATIVE result is that the system is focused on pre-diagnosis, not definitive diagnosis. The severity of the pathologies involved requires confirmation through classical gold-standard methods.

### METHOD OF USE OF THE SAMPLE COLLECTOR

The process of using the exhaled air sample collector follows the steps described below and illustrated in attached Figure 4: the user receives the sealed collector; the user removes the seals and blows into the mouthpiece following the application's step-by-step guidance; the user then reseals the device and sends it for analysis; the collector is transported to the analysis site; the device may be opened to remove the absorbent medium or a capsule containing it, so that laboratory analysis can be carried out using gold-standard equipment or processes (a term commonly used in the medical field to designate the best technological or scientific method available to solve a given problem).

The system and collector, according to the Utility Model, guide the user through an application (Figure 5) regarding the steps to be performed; the system features a mobile phone or smartphone application that, in addition to guiding the step-by-step process, explains the procedure and includes a sound-processing algorithm using the device's microphone, capable of interpreting the power, frequency, and duration of inhalation and exhalation (3). It displays a bar that graphically indicates, by amplitude and color, the intensity and correct duration of each; when approaching the ideal values, the bar turns green, and otherwise, it remains red.

### ARTIFICIAL INTELLIGENCE ANALYSIS SYSTEM

The results of the mass spectrometry analysis may, for example, indicate the presence of organic or inorganic substances, such as ketone, propanol, propanal, pentane, pentanal, methanol, isoprene, cyclohexane, dodecane, butanol, butanone, limonene, benzene, hydroxyacetaldehyde, hydroxy-2-butanone, among others.

These and other biomarkers have been identified and associated with various types of disease, as shown in documents CN114720542A, CN111710372A, WO2014104649A1, WO2022229665A1, CN102326078A, CN112526008A, WO2019053414A1, CN106501430A, WO2017056493A1, CN104919318A, WO2019102221A1, WO2017046182A1, WO2020112027A1, and CN114705777A.

Each biomarker presents a concentration in the sample and may or may not be present, depending on the type of disease and the patient. The presence of such biomarkers is indicative of the occurrence of the disease, and from this information, the physician may request more detailed and invasive tests.

Going beyond simple molecule detection, through machine learning and by correlating this information with patient history and their clinical/behavioral conditions, it is possible to establish parameters for assigning a numerical risk score. This number can be used by physicians to indicate other types of examinations, such as precision MRIs, PET-CT scans, or even biopsies. It can also be used for treatment monitoring, assessing the impact of introducing medications, and conducting periodic tests during treatment and remission. In this way, the complex information from the quantification of biomarkers and their concentration is transformed into a single, simple parameter for faster decision-making, which can, for example, indicate the disease stage.

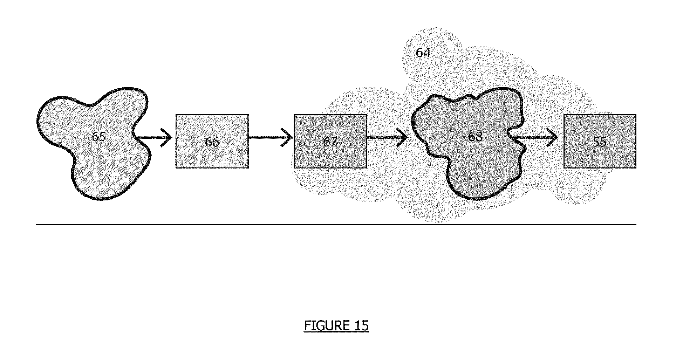

The result processing system is built as described in Figure 15. A machine-learning tool is trained with a dataset (65) of similar characteristics in terms of qualitative and quantitative variation, generating an algorithm (67) capable of processing biomarker data. This algorithm is formatted to interact with the application, thus offering a user-friendly interface (67) for users. The system can then process real data (68) still with a fine-tuning training phase and reduction of false positives and negatives through external monitoring and control. Once false results reach an acceptable level of more than 80% accuracy, the algorithm can be permanently linked to the results organization module (55), making the system 100% functional.

The following are merely illustrative examples of the scope and mechanical details for successive proof-of-concept tests and should not be used for limiting purposes.

### MECHANICAL DESIGN 1

Three types of collectors were used to acquire exhaled air samples from a patient already diagnosed with lung cancer. The first consisted of a laboratory glass tube filled with granulated activated carbon, sealed at the inlet and outlet with sterilized nonwoven fabric (TNT); the second consisted of a laboratory glass flask filled with a solution of distilled water and powdered activated carbon, equipped with a laboratory glass straw; the third consisted of a laboratory glass funnel with walls coated with hydrogel and with the mouth occluded with sterilized nonwoven fabric. All three devices were sealed in an aseptic environment in plastic bags to be transported to the home of the volunteer patient, previously diagnosed with stage 2 lung cancer, finishing hormone therapy for tumor reduction. The collection involved instructing the patient on how to inhale deeply, hold her breath for a few seconds to saturate the inhaled air with VOCs, and blow slowly into the three devices, with the operation repeated three times for each device. After collection, the devices were again sealed in plastic bags and placed inside a box. They were stored for 7 days at the Central Chemical Analysis Laboratory of the Institute of Chemistry of USP before being opened. In the case of the liquid sample, analyses were performed on the lower portion more saturated with powdered activated carbon and on the upper portion with lower concentration; in the case of the tube with solid granules, analyses were performed on the portion closest to the blowing mouthpiece, and the same was done for the hydrogel device. Analyses using the devices with liquid and solid absorption media demonstrated the presence of tumor biomarkers, while the hydrogel result was null, possibly due to the geometry of the test device, which may require improvement, following the schematic models described herein.

Figure 6, attached, represents the result of the analysis of a sample from a patient with lung cancer, using a collector with solid medium according to the Utility Model.

### MECHANICAL DESIGN 2

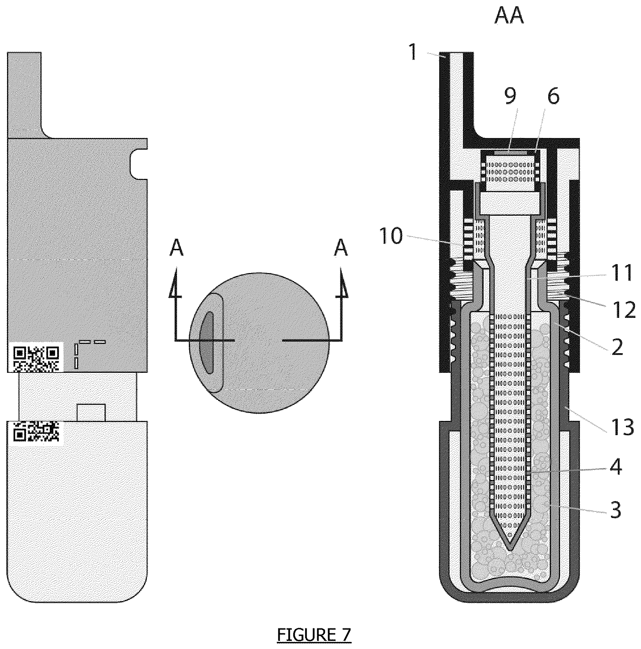

Figure 1 provides a schematic and illustrative form of the concept for understanding the system, showing that biomarkers from the patient's exhalation are collected by passing the air through a chamber with absorbent medium, using barriers at the inlet and outlet to prevent backflow, as well as closure mechanisms for transport and for removing the internal material for analysis, without allowing volatile organic compounds to escape. Figure 7 illustrates the mechanics developed for the device in its version with solid absorbent medium, fulfilling the conceptual function shown in Figure 1, but in a more efficient manner and suitable for the actual process described in Figure 4.

As shown in Figure 7, the device for solid absorbent medium comprises a blowing mouthpiece (1) mounted together with a base (13), protecting an analysis vial (2), sized for the metal trays of common spectrometry equipment (such as GCMS). The vial is made of inert material, not emitting Volatile Organic Compounds (VOCs) at the temperature of use and analysis (100 degrees Celsius), and may be made of glass, metal, or thermoplastic resins (polycarbonate, polypropylene, or others), sterilizable for reuse or recyclable. Inside the assembly there is a movable needle (11) with an upper cap (6) having a weakened region (9) created by thickness reduction or by deposition of a thermoplastic rubber membrane. The interface between the blowing mouthpiece (1) and the base (13) features a thread (12) that allows assembly, controlled variation of the distance between them, and also sealing of the set after collection, as explained below.

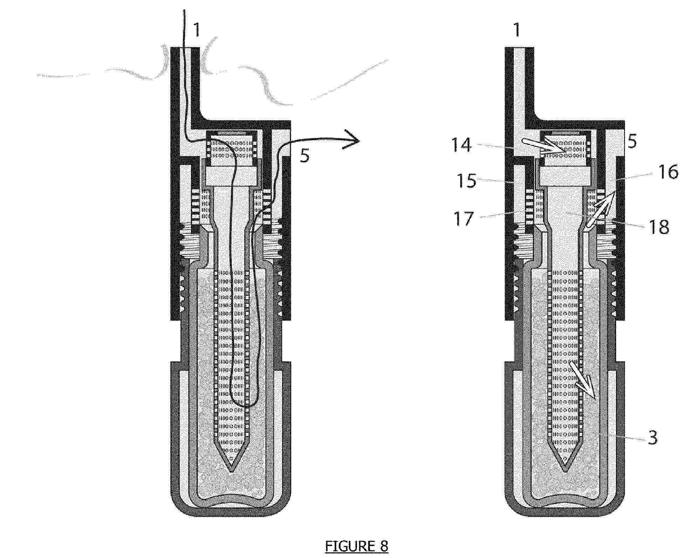

In Figure 8, so that the air exhaled by the patient through the mouthpiece opening (1), enriched with volatile biomarkers, follows a path through the absorbent medium and is expelled through the outlet opening (5), the needle and its cap have passage holes, as does the inner skirt of the blowing mouthpiece (17). The holes allow the passage of air and prevent the return or exit of the solid absorbent material in granules, which, in the case of Proof of Concept 2, are coconut-fiber activated carbon grains with high porosity and purity, particle size of 1 to 2 mm. It is desirable that the absorbent medium be mono-material, so as not to generate interference in the detected molecular spectrum, and neutral so as not to interact with the Volatile Organic Compounds (VOCs) or the patient's health. As shown in Figure 8, the exhaled air enters through the opening (1) and fills the first cavity (14). The exposed holes of the needle cap allow passage to the second cavity (18), the interior of the needle. The perforations in the needle (4) allow the air to pass through the absorbent medium (3) and then to the next cavity in the circuit (15/16), which communicates with the exterior through the air outlet opening (5).

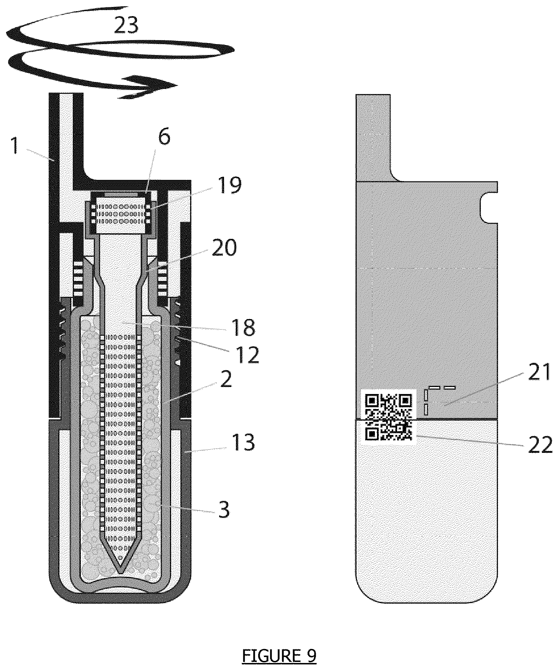

Figure 9 shows the sealing and protection operation of the vial for transport to the laboratory, immediately after the sample collection performed by the patient. By rotating (23) the blowing mouthpiece (1), the thread between it and the base (12) draws them closer together, pressing the cap (6) against the needle, which also moves downward, embedding into the neck of the vial (20). This reconfiguration prevents the absorbent medium (3) from contacting the external environment, preserving the volatile biomarkers inside the vial, sealed jointly by the cap (6) and the needle (18), through radial pressure and contact between surfaces (19 and 20). At the end of the rotation stroke, there is a seal (21) with tactile feedback for greater sample and patient safety, preventing reverse rotation unless the seal wall is broken with a tool or by reverse rotation using force. There is also a visual code which, in full closure, has the two halves coinciding, allowing the application to recognize that the closure was properly performed.

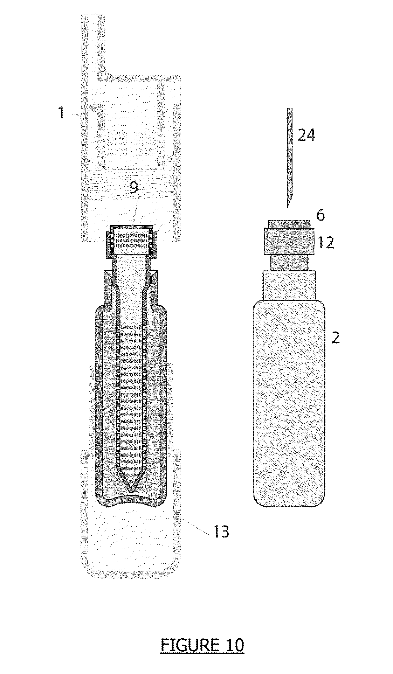

Figure 10 shows the opening of the assembly for laboratory analysis. This is done by unscrewing the blowing mouthpiece (1) from the base (13), breaking the seal (21 of Figure 9) and granting access to the vial (2), which is occluded by the needle assembly (18) and cap (9), so that it can be removed from the base. To conduct the analysis, it is not necessary to remove this assembly, as the spectrometer sampling needle (24) can be inserted directly through the weakened area of the cap (9) without exposing the internal environment saturated with volatile biomarkers to external air.

### MECHANICAL DESIGN 3

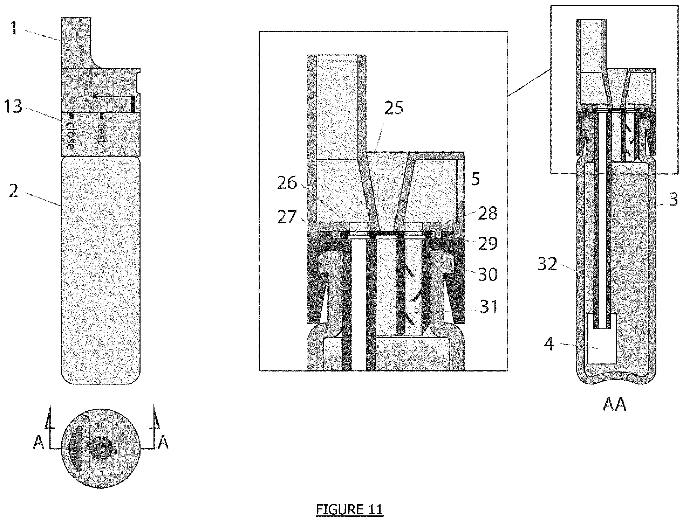

Figure 11 presents the version of the system designed for multi-media, which may be liquids, solids, and gels. Following the concept schematically shown in Figure 1, in which the absorption of volatile organic components occurs through the passage of exhaled air from the patient through a chamber with absorbent medium, the device in this version comprises a vial (2) made of glass, metal, or thermoplastic resins (polycarbonate, polypropylene, or others), sterilizable for reuse or recyclable, manufactured from inert material that does not emit Volatile Organic Compounds (VOCs) at the temperature of use and analysis (100 °C). Over it is applied a cap composed essentially of two main parts: the rotatable blowing mouthpiece (1) mounted on the base (13). The cross-section view reveals the internal structures: inlet air-passage opening (26) from the blowing mouthpiece to the cannula (32), which directs the exhaled air, rich in volatile biomarkers, to the atomizer or micro-bubble generator (4) and reverse-flow retainer, which may be made of sintered ceramic or high-porosity polymers; rotation ring and connection between blowing mouthpiece and base (27); deflection duct to prevent the exit of liquid or gel (31); liquid, gel, or solid medium (3); flange for retaining the cap on the vial neck (30); thermoplastic rubber piece for compression sealing (29), a classic hydraulic sealing solution with O-rings; outlet air-passage opening from the absorbent cavity (28); and air-outlet opening from the device (5).

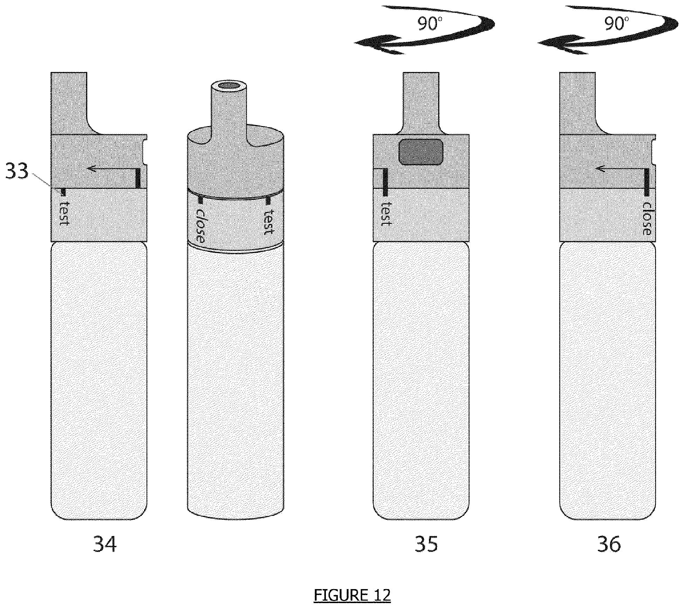

Figure 12 presents the operational process of the device in three stages: closed before testing (34), open for testing (35), and closed again for transport and analysis (36). The transition between each of these three stages is carried out by rotating the blowing mouthpiece clockwise in 90-degree steps, aided by a tactile marker and by mechanical stops present in the rotation ring. These structures prevent a return to the open position when the patient closes the system immediately after the test and ensure the integrity of the sample, aligned with the visual marking for the patient (33).

Figure 13 further details the open (39) and closed (37/38) states. The opening holes of the thermoplastic rubber piece (29) responsible for sealing (26/28) are offset by 90 degrees from the air-passage holes in the base (45/46), thereby occluding the air passage (40/41). By rotating the blowing mouthpiece (39), the holes align, unblocking the air passage for the testing moment. To close the device after testing, the user simply rotates it another 90 degrees clockwise so that misalignment and occlusion occur again (40/41), preserving the sample. In the open state (39), the blown air (48) passes through the blowing mouthpiece (1), fills the cavity (14), passes through the passage holes (45 aligned with 26), enters the absorption cavity via the cannula (42) reaching the atomizer or micro-bubble generator (4), passes (51) into the absorbent medium (3), flows vertically (50), passes through the passage holes to the outlet (46 aligned with 28), and finally leaves (49) the device through the outlet opening (5).

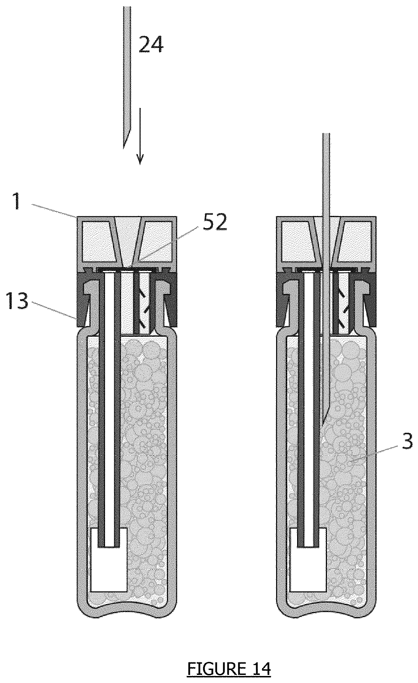

Figure 14 presents the analysis moment. The device allows the usual method used in spectrometers, which consists of inserting a needle (24) through region (52) of the protective rubber membrane to collect the sample. The device also allows complete removal of the cap formed by the base and blowing mouthpiece (1) to access the absorbent medium (3), or the use of a syringe to remove it.

## Claims

1. Breath sample collector for the pre-diagnosis of pathologies, **characterized in that** said portable, disposable or returnable collector comprises: blowing mouthpiece (1); collection chamber (2); solid and/or liquid and/or gel internal medium for absorption of volatile or non-volatile substances (3); valve systems (4) that allow air passage but block the return of air from the absorption medium; air outlet (5); air outlet occlusion (6); blowing mouthpiece occlusion (7); occlusion preventing accidental removal of the absorption medium (8) so that the samples are obtained and preserved; final seal (21) with tactile feedback and a visual code indicating complete closure.

2. Breath sample collector for the pre-diagnosis of pathologies, according to claim 1, **characterized in that** the collector is a compact portable device equipped with multiple chambers and compatible with multiple absorption media, disposable or returnable, comprising: blowing mouthpiece (1); collection chamber(s) (2); solid and/or liquid and/or gel internal medium for absorption of volatile organic substances (3); valve (4) that allows exhaled air passage but blocks the return from the absorption medium; air outlet (5); sealing and closure systems for sample preservation, prevention of improper removal of the absorption medium, and prevention of contamination (6/7/8), so that biomarker samples related to the pathologies are collected and sealed easily and intuitively by the patient themself (36), with visual and tactile feedback assistance (21), outside clinical or laboratory environments and without the need for healthcare professionals, ensuring they are properly protected for transport, temporary storage, safe access (5), and remote processing in a specialized laboratory (24), providing raw data for interpretation by an artificial intelligence algorithm (68) that delivers a risk level as pre-diagnostic information for medical or public health decisions, aiming at patient monitoring or further investigation.

3. Breath sample collector for the pre-diagnosis of pathologies, according to claim 1, **characterized in that** the breath sample collector may be of four types, comprising: liquid absorbent medium, or solid absorbent medium, solid and liquid absorbent medium (hybrid), or compatible with multiple absorbent media.

4. Breath sample collector for the pre-diagnosis of pathologies, according to claim 1, **characterized in that** the collector may internally comprise multiple internal chambers with absorption media that capture volatile substances from the exhaled air.

5. Breath sample collector for the pre-diagnosis of pathologies, according to claim 1, **characterized in that** the internal medium comprises a medium selected from the group consisting of: distilled water with absorbent solutes, powdered or granulated activated carbon, hydrogel, aerogel, calcium hydroxide, sodium hydroxide, graphene sheets, MOFs (metal-organic frameworks), and porous polymers.

6. Breath sample collector for the pre-diagnosis of pathologies, according to claim 1 or 3, **characterized in that** the collector with liquid or gel absorbent medium comprises: blowing mouthpiece (1); collection chamber (2); internal liquid or gel medium for absorption of Volatile Organic Compounds (VOCs) (3); valve (4) that allows air passage but blocks the return from the absorbent medium; air outlet (5); cap for the air outlet (6); cap for the blowing mouthpiece (7); and cap for removal of the absorbent medium (8) so that the samples can be obtained.

7. Breath sample collector for the pre-diagnosis of pathologies, according to claim 1, **characterized in that** the collector with hybrid absorbent medium (solid and/or liquid and/or gel) comprises: blowing mouthpiece (1); collection chambers (2); solid absorption medium (3A); liquid or gel absorption medium (3B); and possibly additional passage chambers beyond these two, in order to maximize sampling; valve (4) that allows gas passage but blocks return from the liquid, gel, or solid absorption medium; air outlet (5); air outlet cap (6); blowing mouthpiece cap (7); cap for removal of the absorption medium (8) to obtain the samples.

8. Breath sample collector for the pre-diagnosis of pathologies, according to claim 1 or 3, **characterized in that** the collector with hybrid absorbent medium (solid and/or liquid and/or gel) comprises: blowing mouthpiece (1); collection chambers (2); solid absorbent medium (3A); liquid or gel absorbent medium (3B); valve (4) that allows gas passage but blocks the return from the liquid, gel, or solid absorbent medium; air outlet (5); cap for the air outlet (6); cap for the blowing mouthpiece (7); and cap for removal of the absorbent medium (8) so that the samples can be obtained.

9. Breath sample collector for the pre-diagnosis of pathologies, according to claim 1, **characterized in that** the multi-media collector comprises a blowing mouthpiece (1) with a sealing and locking system for sample preservation (45/46/26/28), an air outlet (5), a containment container (2) for more than one type of absorbent medium (3) which is the analysis vial itself and therefore does not require an external casing, and a non-return filter for the absorbent media to prevent their return to the mouthpiece (4).

10. Breath sample collector for the pre-diagnosis of pathologies, according to claim 1, **characterized in that** the collector for solid absorbent medium comprises a blowing mouthpiece (1) mounted together with a base (13), protecting an analysis vial (2), a movable needle (11) having an upper cap (6) provided with a weakened region (9) by thickness reduction or by deposition of a thermoplastic rubber membrane, wherein the interface between the blowing mouthpiece (1) and the base (13) is provided with a thread (12) that allows assembly, controlled variation of the distance between them, and also sealing of the set after collection.

11. Breath sample collector for the pre-diagnosis of pathologies, according to claim 1, **characterized in that** the needle and its cap are provided with passage perforations, as is the inner skirt of the blowing mouthpiece (17), wherein the air exhaled by the patient through the mouthpiece opening (1) fills the first cavity, the exposed holes of the needle cap allowing its passage to the second cavity (18) inside the needle, the air then passing through the absorbent medium (3) and subsequently to the next cavity in the circuit (15/16), which communicates with the exterior through the air outlet opening (5).

12. Breath sample collector for the pre-diagnosis of pathologies, according to claim 1, **characterized in that** rotation (23) of the blowing mouthpiece (1) brings the thread between it and the base (12) closer together, pressing the cap (6) against the needle (18), which also moves downward, embedding into the interior of the vial neck (20), wherein at the end of the stroke there is a seal (21) with tactile feedback and a visual code indicating complete closure.

13. Breath sample collector for the pre-diagnosis of pathologies, according to claim 1, **characterized in that** the multi-absorbent media collector comprises: a glass vial (2), over which is applied a cap composed essentially of two main parts: the rotatable blowing mouthpiece (1) mounted on the base (13), wherein the inlet air-passage opening (26) from the blowing mouthpiece to the cannula (32) directs the exhaled air, rich in volatile biomarkers, to the atomizer or micro-bubble generator (4) and reverse-flow retainer; rotation ring and connection between blowing mouthpiece and base (27); deflection duct to prevent the exit of liquid or gel (31); liquid, gel, or solid medium (3); flange for retaining the cap on the vial neck (30); thermoplastic rubber piece for compression sealing (29), a classical hydraulic sealing solution with O-rings; outlet air-passage opening from the absorbent cavity (28); and air outlet opening from the device (5).

14. Method of use of the breath sample collector, according to any of the preceding claims, **characterized in that** the user receives the sealed collector; the user removes the seals and blows into the mouthpiece following the step-by-step instructions of the application; the user then reseals the device and sends it for analysis; the collector is transported to the analysis site; the device may be opened to remove the absorbent medium or a capsule containing it, so that the laboratory analysis process can be carried out using gold-standard equipment or processes (a term commonly used in the medical field to designate the best technological or scientific method available for solving a given problem).

15. Method of use of the breath sample collector, according to claim 14, **characterized in that** the biomarkers captured in the collector are analyzed by mass spectrometry or another device/process capable of providing the same level of reliability, comprising hardware with an electronic nose (a sensor capable of identifying gaseous molecules in a sample), an electrical spectrum analyzer for solutions (identifying molecules by changes in electrical current in a solution), or other molecular analysis technologies, or those that can be miniaturized and made inexpensive enough to be integrated into the solution, thus being able to indicate the presence of organic substances such as ketone, propanol, propanal, pentane, pentanal, methanol, isoprene, cyclohexane, dodecane, butanol, butanone, limonene, benzene, hydroxyacetaldehyde, hydroxy-2-butanone, among others.

16. Method of use of the breath sample collector, according to claim 14, **characterized in that** the patient blows into the portable breath sample collector from the lungs for capturing volatile organic compounds (VOCs), being guided by a mobile phone or smartphone application that provides step-by-step instructions for using the portable collector and is capable of measuring, through sound signal filtering and/or artificial intelligence, the effectiveness, duration, and strength of the blow, providing real-time information on the effectiveness of the collection; upon completion of the procedure, the collector is stored and transported to the analysis site, where molecular analysis of the captured VOCs is carried out to identify pathology biomarkers, which may be assisted by artificial intelligence combined with the patient's profile to identify and determine the risk level or disease stage.

17. Method of use of the breath sample collector, according to claim 14, **characterized in that** the user is guided in the use of the collector through an application regarding the steps to be performed; the mobile phone or smartphone application, in addition to guiding the step-by-step process, explains the procedure and includes a sound-processing algorithm using the device's microphone to interpret the power, frequency, and duration of inhalation and exhalation (3), displaying a bar that graphically indicates, by amplitude and color, the intensity and correct duration thereof, turning green when close to the ideal values, and remaining red otherwise.

18. Method of use of the breath sample collector, according to claim 14, **characterized in that** the application explains how the capture is performed and how the system works; instructs the user on how to handle and use the device; assists the user in inhaling and exhaling correctly to maximize test success; and informs how to seal the sample inside the device and how to send it for analysis, so that the pre-diagnosis can be carried out remotely by individuals without intensive training and without investment in equipment.

19. Method of use of the breath sample collector, according to claim 18, **characterized in that** the pathology pre-diagnosis system is assisted by a smartphone application, which, in addition to guiding the collection procedure step-by-step, also assists the user in maximizing inhalation and exhalation to provide the best possible sample, measuring their intensity and duration through sensing, and displaying this through graphical resources.

20. Method of use of the breath sample collector, according to claim 14, **characterized in that** it comprises the stages: closed before testing (34), open for testing (35), and closed again for transport and analysis (36), wherein the transition between each of these three stages is carried out by rotating the blowing mouthpiece clockwise in 90-degree steps, aided by a tactile marker and also by mechanical stops in the rotation ring.

21. Method of use of the breath sample collector, according to claim 14, **characterized in that** a machine learning tool is trained with a dataset (65) with similar characteristics in terms of qualitative and quantitative variation, generating an algorithm (67) capable of processing the biomarker data, said algorithm being formatted to interact with the application and thus provide a user-friendly interface (67) for the system to begin processing real data (68), still in a fine-tuning phase and reducing false positives and negatives through external monitoring and control, wherein once false results reach an acceptable level of more than 80% accuracy, the algorithm can be permanently connected to the results organization module (55), making the system 100% functional.
